# EUROPEAN PATENT SPECIFICATION

(11) **EP 3 998 985 B1**
(45) Date of publication and mention of the grant of the patent: **30.08.2023**
(21) Application number: 20746715.0
(22) Date of filing: 20.07.2020
(51) Int. Cl.: A61C 7/00

(54) **VIRTUAL ARTICULATION IN ORTHODONTIC AND DENTAL TREATMENT PLANNING**
VIRTUELLES GELENK IN DER ORTHODONTISCHEN UND ZAHNÄRZTLICHEN BEHANDLUNGSPLANUNG
ARTICULATION VIRTUELLE EN PLANIFICATION DE TRAITEMENT ORTHODONTIQUE ET DENTAIRE

(30) Priority: 18.07.2019 US 201962875866 P
(43) Date of publication of application: 25.05.2022
(73) Proprietor: 3M Innovative Properties Company, Saint Paul, Minnesota 55133-3427 (US)
(72) Inventor: SOMASUNDARAM, Guruprasad, Saint Paul, Minnesota 55133-3427 (US); CUNLIFFE, Alexandra R., Saint Paul, Minnesota 55133-3427 (US); ZIMMER, Benjamin D., Saint Paul, Minnesota 55133-3427 (US); STANKIEWICZ, Brian J., Saint Paul, Minnesota 55133-3427 (US); BEN-GAL NGUYEN, Nitsan, Saint Paul, Minnesota 55133-3427 (US); RABY, Richard E., Saint Paul, Minnesota 55133-3427 (US); ALVAREZ, Alberto, 28027 Madrid (ES)
(74) Representative: Vossius & Partner Patentanwälte Rechtsanwälte mbB
(86) International application number: PCT/IB2020/056799
(87) International publication number: WO 2021/009733

(56) References cited:
- WO-A1-2020/026117
- US-A1- 2002 064 746
- US-A1- 2011 270 588
- US-A1- 2017 273 759

## Description

### TECHNICAL FIELD

This disclosure relates to dental treatment planning using virtual articulation.

### BACKGROUND

The field of orthodontics relates to repositioning a patient's teeth for improved function and aesthetic appearance. Orthodontic devices and treatment methods generally involve the application of forces to move teeth into a proper bite configuration, or occlusion. As one example, orthodontic treatment may involve the use of slotted appliances, known as brackets, which are fixed to the patient's anterior, cuspid, and bicuspid teeth. An archwire may be placed in the slot of each bracket and serves as a track to guide movement of the teeth to desired orientations. The ends of the archwire are received in appliances known as buccal tubes that are secured to the patient's molar teeth. Such dental appliances remain in the mouth of the patient and are periodically adjusted by an orthodontist until proper alignment is achieved.

Orthodontic treatment may also involve the use of alignment trays, such as clear or transparent, polymer-based tooth positioning trays, often referred to as clear tray aligners (CTAs). For example, orthodontic treatment with CTAs may include forming a tray having shells that engage one or more teeth. Each shell may have a shape that is deformed upon being installed over the patient's teeth. The deformed position of a respective shell of the CTA may apply a force to a respective tooth toward a desired position of the tooth that is an intermediate position between an initial position of the respective tooth and a final position resulting from the orthodontic treatment. However, orthodontic treatment may require some tooth movements that are difficult for a CTA to achieve, such as, for example, tooth root movements and rotations of cuspids and bicuspids. In these instances, the forces and moments that a CTA is capable of applying directly to the surfaces of a tooth may be insufficient to achieve the desired tooth movement.

Digital dentistry is a growing trend with an increasing number of dentists using digital impressioning systems. These systems use an intra-oral scanning camera, or scanning of a traditional physical impression, and an associated processing system to generate a digital three-dimensional (3D) model of patients' teeth (e.g., a patient's maxillary and mandibular arches). The digital 3D models can then be used to make prosthodontic restorations and for orthodontic treatment planning.

The goal of the orthodontic treatment planning process is to determine where the posttreatment positions of a person's teeth (setup state) should be, given the pre-treatment positions of the teeth in a malocclusion state. This process is typically performed manually using interactive software and is a very time-consuming process. Intermediate staging of teeth from a malocclusion state to a final state may include determining accurate individual teeth motions in a way that teeth are not colliding with each other, the teeth move toward their final state, and the teeth follow an optimal (preferably short) trajectories. Since each tooth has 6 degrees-of-freedom and an average arch has about 14 teeth, finding the optimal teeth trajectory from initial to final stage has a large and complex search space.

Accurate articulation is one factor in making such orthodontic treatment plans. Current data acquisition for mechanical articulation is time consuming and requires expensive analog devices. In particular, current example techniques involve a manual process using a face bow and lab articulator to capture mandibular articulation data for complex rehabilitations.

US 2002/064746 A1 relates to systems and methods to prepare a malocclusion treatment plan by selecting a tooth treatment pattern from a library of predetermined tooth treatment patterns, and generating the malocclusion treatment plan implementing the selected tooth treatment pattern.

US 2017/273759 A1 relates to an apparatus and method defining a fit of a set of upper and lower teeth of a patient by generating a computer representation of the teeth, and determining an occlusion from the computer representation of the teeth using one or more keys

### SUMMARY

The invention is defined by the features of the independent claims. Preferred embodiments are defined in the dependent claims.

In general, this disclosure describes a computer-implemented method and system for evaluating, modifying, and determining setups for orthodontic treatment using metrics computed during virtual articulation. Virtual articulation may refer to the measurement and/or visualization of how three-dimensional scans of a patient's teeth (e.g., the mandibular arch and the maxillary arch) interact with each other at one or more stages of a treatment plan for the purpose of determining a final orthodontic treatment and other dental health planning. The virtual articulation techniques of this disclosure may further be combined with techniques for determining dynamic collision metrics, determining a comfort measurement, determining treatment efficacy, and/or determining dental conditions, such as bruxism. The techniques of this disclosure further include user interface techniques that provide an orthodontist/dentist/technician with information regarding various treatment plans based on metrics gathered during virtual articulation.

In some examples, this disclosure describes a fully automated approach to create intermediate states (i.e., arrangements of teeth) that enable a valid trajectory from a maloccluded state (e.g., an initial state) to a setup state (e.g., a final state). The techniques and systems of this disclosure may be used in conjunction with orthodontic treatment planning for clear tray aligners as well as other treatment modalities (e.g., the Incognito^{™} Appliance System made by 3M, brackets and wires, etc.) or even multi-modality treatment. Elements of this disclosure may also be used for the visualization or prediction of tooth movement in digital dentistry. The techniques of this disclosure may be implemented in a fully automated treatment planning system or in an interactive software system used by a technician or orthodontist who is designing the treatment plan.

The techniques and systems of this disclosure may enable several improvements over the current treatment planning approaches, including improved scalable clear tray aligner throughput and sales, the ability to generate multiple options to present to a doctor and patient, ability for a doctor to review final setup and intermediate setups together (from a two-phased to one-phased approach), and/or the ability to incorporate increased complexity introduced by biomechanics-based rules and/or complicated, multi-appliance treatments.

In one example, this disclosure describes a method comprising receiving, by a computing device, data indicative of a scan of a virtual maxillary arch representing a maxillary arch of a patient and a virtual mandibular arch representing a mandibular arch of the patient, determining one or more treatment plans for the patient based on an initial state of the virtual maxillary arch and the virtual mandibular arch, modifying, by the computing device, the virtual maxillary arch and the virtual mandibular arch to generate a target state for each of the one or more treatment plans, virtually articulating, by the computing device, the modified virtual maxillary arch and the modified virtual mandibular arch to determine contact points at the target state for each of the one or more treatment plans, computing, by the computing device, a dynamic collision metric based on the contact points at the target state for each of the one or more treatment plans, and outputting, by the computing device, data indicative of the dynamic collision metric for each of the one or more treatment plans.

In another example, this disclosure describes a system comprising a memory configured to store data indicative of a scan of a virtual maxillary arch representing a maxillary arch of a patient and a virtual mandibular arch representing a mandibular arch of the patient, and a processor in communication with the memory, the processor configured to determine one or more treatment plans for the patient based on an initial state of the virtual maxillary arch and the virtual mandibular arch, modify the virtual maxillary arch and the virtual mandibular arch to generate a target state for each of the one or more treatment plans, virtually articulate the modified virtual maxillary arch and the modified virtual mandibular arch to determine contact points at the target state for each of the one or more treatment plans, compute a dynamic collision metric based on the contact points at the target state for each of the one or more treatment plans, and output data indicative of the dynamic collision metric for each of the one or more treatment plans.

In another example, this disclosure describes a non-transitory computer-readable storage medium storing instructions that, when executed, cause at least one processor to receive data indicative of a scan of a virtual maxillary arch representing a maxillary arch of a patient and a virtual mandibular arch representing a mandibular arch of the patient, determine one or more treatment plans for the patient based on an initial state of the virtual maxillary arch and the virtual mandibular arch, modify the virtual maxillary arch and the virtual mandibular arch to generate a target state for each of the one or more treatment plans, virtually articulate the modified virtual maxillary arch and the modified virtual mandibular arch to determine contact points at the target state for each of the one or more treatment plans, compute a dynamic collision metric based on the contact points at the target state for each of the one or more treatment plans, and output data indicative of the dynamic collision metric for each of the one or more treatment plans.

The details of one or more aspects of the disclosure are set forth in the accompanying drawings and the description below. Other features, objects, and advantages will be apparent from the description and drawings, and from the claims.

### BRIEF DESCRIPTION OF DRAWINGS

FIG. 1 is a block diagram illustrating an example system for virtual articulation according to one example of the disclosure.
FIG. 2 illustrates an example of a digital 3D model of a patient's teeth.
FIG. 3 illustrates an example user interface showing contact maps.
FIG. 4 illustrates another example user interface showing contact maps with teeth movement.
FIG. 5 illustrates another example user interface showing collision metrics and discomfort scores at various states.
FIG. 6 illustrates another example user interface comparing a patient's metrics with population data metrics.
FIG. 7 is flow diagram showing one example process using the techniques of this disclosure.
FIG. 8 is a flowchart showing example operation of a virtual articulation system according to techniques of this disclosure.

### DETAILED DESCRIPTION

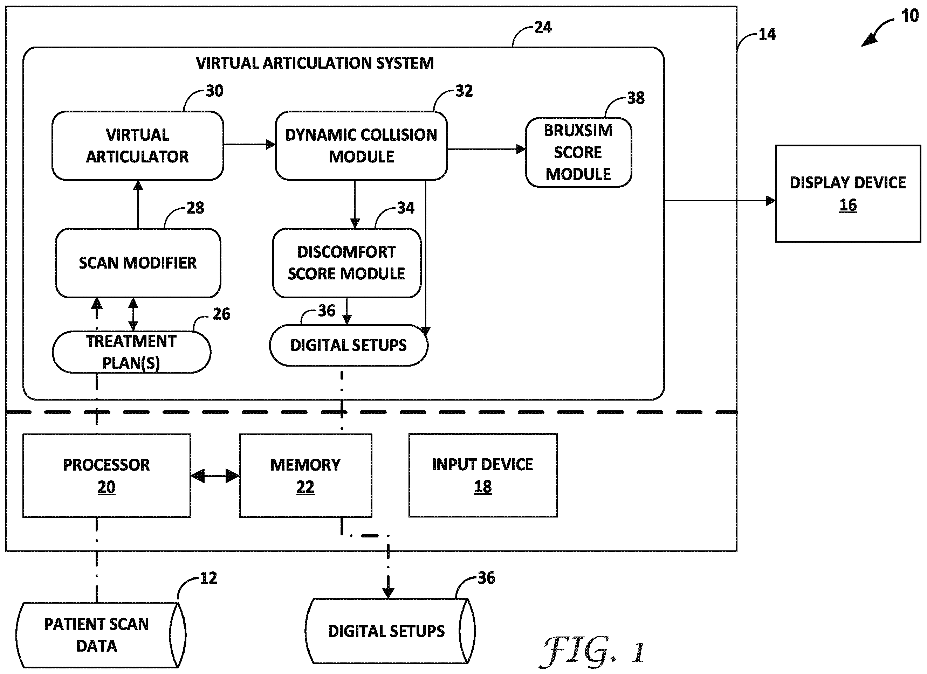

FIG. 1 is a diagram of an example system 10 for performing virtual articulation and computing metrics from the virtual articulation using digital 3D models from intra-oral scans of a patient. System 10 can be implemented with, for example, a desktop computer, notebook computer, tablet computer, or any type of computing device. System 10 includes a computer 14 configured to receive patient scan data 12 and store patient scan data 12 in memory 22. Patent scan data 12 may include digital 3D models of teeth or other intra-oral structures from intra-oral 3D scans or scans of impressions or castings of teeth. In some examples, patient scan data 12 may include scans of the mandibular arch (e.g., lower jaw and teeth) and the maxillary arch (e.g., upper jaw and teeth) of a patient.

Patient scan data 12 may comprise 3D models of the mandibular arch and maxillary arch of a patient. The use of digital 3D models in the dental market is becoming more prevalent. In one example, patient scan data 12 can be acquired directly in vivo using an intra-oral scanner, Cone Beam Computed Tomography (CBCT) scanning (i.e., 3D X-ray), or Magnetic Resonance Imaging (MRI). In other examples, patient scan data 12 can be acquired indirectly by scanning an impression of the teeth or a casting made from an impression of the teeth. Some examples of indirect data acquisition methods include, but are not limited to, industrial Computed Tomography (CT) scanning (i.e., 3D X-ray), laser scanning, and patterned light scanning. Patient scan data 12 can be used for varied clinical tasks including treatment planning, crown and implant preparation, prosthodontic restorations, orthodontic setup design, orthodontic appliance design, and in diagnostic aides, for example to assess or visually illustrate tooth wear. As will be explained in more detail below, system 10 may use patient scan data 12 to perform virtual articulation at one or more stages of a dental treatment plan, calculate dynamic collision metrics based on the virtual articulation, and output the data indicative of the dynamic collision metrics in a way that allows a user to determine the efficacy of dental treatment plans, select particular dental treatment plans, and/or modify a dental treatment process.

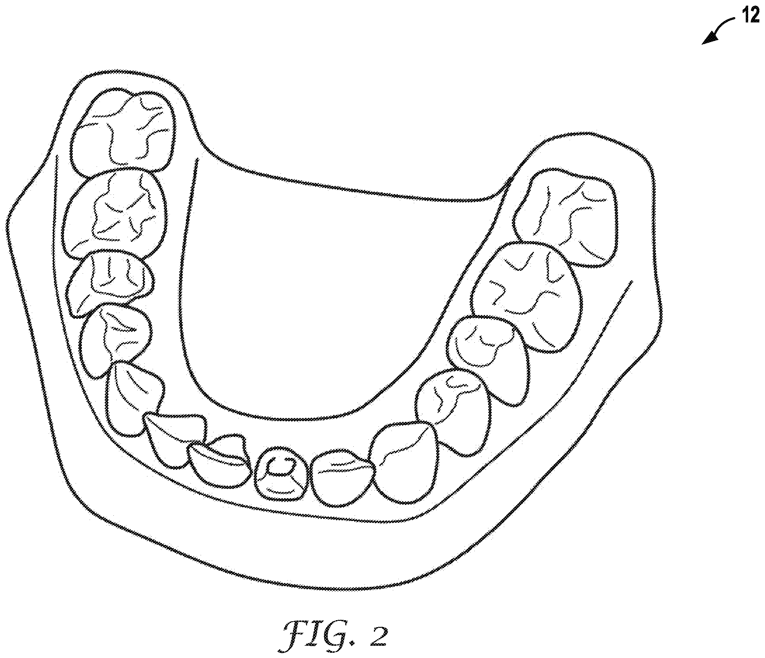

An example of a digital 3D model of a patient's mandibular arch (e.g., patient scan data 12) from a scan is shown in FIG. 2. A similar scan may be made of a patient's maxillary arch. The scans of a patient's mandibular arch and maxillary arch may be referred to as a virtual mandibular arch and a virtual maxillary arch, respectively. Systems to generate digital 3D images or models based upon image sets from multiple views are disclosed in U.S. Patent Nos. 7,956,862 and 7,605,817. These systems can use an intra-oral scanner to obtain digital images from multiple views of teeth or other intra-oral structures, and those digital images are processed to generate a digital 3D model or scan representing the scanned teeth or other intra-oral structure. The 3D models or scans can be implemented as, for example, a polygonal mesh or point cloud representing the surface of the scanned object or intra-oral structure.

Intra-oral structures include dentition, and more typically human dentition, such as individual teeth, quadrants, full arches, pairs of arches which may be separate or in occlusion of various types, soft tissue (e.g., gingival and mucosal surfaces of the mouth, or perioral structures such as the lips, nose, cheeks, and chin), and the like, as well as bones and any other supporting or surrounding structures. Intra-oral structures can possibly include both natural structures within a mouth and artificial structures such as dental objects (e.g., prosthesis, implant, appliance, restoration, restorative component, or abutment).

Returning to FIG. 1, system 10 may also include an electronic display device 16 for displaying digital 3D models from scans of intra-oral structures. In some examples, display device 16 is part of computer 14, and in other examples, display device 16 may be separate from computer 14. Display device 16 can be implemented with any electronic display, for example a Cathode Ray Tube (CRT), a liquid crystal display (LCD), light emitting diode (LED) display, or organic light emitting diode (OLED) display.

System 10 may further include an input device 18 for receiving user commands or other information. In some examples, input device 18 is part of computer 14, and in other examples, input device 18 may be separate from computer 14. Input device 18 can be implemented with any device for entering information or commands, for example a keyboard, microphone, cursor-control device, or touch screen. The components of system 10 may also be combined, e.g., a tablet computer can incorporate the processor, display and touch screen input devices into a single unit.

Intermediate staging of teeth from a malocclusion state to a final state includes determining accurate individual teeth motions in such a way that teeth have an acceptably low amount of collision with each other, the teeth move toward their final state, and the teeth follow optimal (preferably short) trajectories. Since each tooth has 6 degrees-of-freedom and an average arch has about 14 teeth, finding the optimal teeth trajectory from initial to final stage has a large and complex search space. An orthodontist may define a treatment plan that defines a target final state of the patient's teeth. The treatment plan may also define one or more desired intermediate states of the teeth as well as the treatment modalities used to achieve the target final state.

System 10 may be configured to receive one or more treatment plans 26. In some examples, a user (e.g., orthodontist) may input treatment plans to computer 14 using input device 18. Computer system 14 may store treatment plans 26 in memory 22. In some examples, treatment plans 26 may include an initial state of the virtual maxillary arch and the virtual mandibular arch as well as a target state (e.g., the final position after treatment) for the patient's teeth. Using the techniques of this disclosure described below, system 10 may perform virtual articulation to determine the efficacy of the target state for treatment plans 26. System 10 may also be configured to determine one or more intermediate states to include in treatment plans 26. In other examples, system 10 or a user may not determine intermediate states until the efficacy and desirability of the target final state is determined.

In other examples, treatment plans 26 may include one or more intermediate states as well as a target final state. Using the techniques of this disclosure described below, system 10 may perform virtual articulation at each of these states to determine the efficacy of the target state for treatment plans 26.

Processor 20 may be configured to use patient scan data 12 and treatment plans 26 to perform virtual articulation and calculate metrics in accordance with the techniques of this disclosure. In the example of FIG. 1, processor 20 is configured to execute code for virtual articulation system 24 to perform the techniques of this disclosure. The techniques described herein can be implemented in software or firmware modules, for example, for execution by processor 20 or other computing devices. In other examples, the techniques of this disclosure may be implemented in hardware modules or a combination of software and hardware.

In various examples, processor 20 may include, be, or be part of programmable processing circuitry, fixed function circuitry, one or more processors, such as one or more digital signal processors (DSPs), general purpose microprocessors, application specific integrated circuits (ASICs), field programmable gate arrays (FPGAs), or other equivalent integrated or discrete logic circuitry, as well as any combination of such components.

In the example of FIG. 1, virtual articulation system 24 may include scan modifier 28, virtual articulator 30, dynamic collision module 32, discomfort score module 34, and bruxism score module 38. The modules shown in FIG. 1 are just examples. The techniques of each of the aforementioned modules may be combined or separated into any number of software modules.

Scan modifier 28 may be configured to receive patient scan data 12 and treatment plans 26. As discussed above, in some examples, treatment plans 26 may define a desired final state of the patient's teeth. In other examples, treatment plans 26 may define one or more intermediate states of the patient's teeth as well as a desired final state of the teeth.

Scan modifier 28 may be configured to extract the state information for each of treatment plans 26 and modify the virtual maxillary arch and the virtual mandibular arch of patient scan data 12 at the target state for each of the one or more treatment plans 26. If treatment plans 26 include intermediate states, scan modifier 28 may be further configured to modify the virtual maxillary arch and the virtual mandibular arch of patient scan data 12 at each of the intermediate states for each of the one or more treatment plans 26. Scan modifier 28 may modify the virtual maxillary arch and the virtual mandibular arch to match the teeth position at each of the states of treatment plans 26.

Virtual articulator 30 may receive the modified virtual maxillary arch and the modified virtual mandibular arch for each of treatment plans 26 and perform virtual articulation on the modified scans. In general, virtual articulation may involve virtually moving the modified scans through various mandibular motions to simulate how a patient's teeth interact in different states during the treatment process. In one example, virtual articulator 30 may articulate the modified virtual maxillary arch and the modified virtual mandibular arch to determine contact points of the patient's teeth at the target state for each of the one or more treatment plans 26. In other examples, virtual articulator 30 may articulate the modified virtual maxillary arch and the modified virtual mandibular arch to determine contact points of the patient's teeth at one or more intermediate states and at the target state for each of the one or more treatment plans 26.

Virtual articulator 30 may be configured to move the modified virtual maxillary arch and the modified virtual mandibular arch through various mandibular poses to simulate a normal range of motion of a patient's teeth. Example mandibular poses may include motions, including one or more of a protrusive excursion, a retrusive excursion, a left lateral excursion, or a right lateral excursion.

Using the virtual maxillary arch as a fixed reference coordinate system, virtual articulator 30 may be configured to transform the relative relationships between the virtual maxillary arch and the virtual mandibular arch into a shared coordinate system to attain transforms describing the various mandibular poses for each individual type of articulation relative to a closed pose, in particular closed to open, closed to protrusive, closed to lateral left, and closed to lateral right. Various forms of interpolation of the mandible position of the virtual mandibular arch and orientation between the closed and respective bite pose, reflecting the mandible motion to attain that specific pose, are then possible. The overall mandibular motion in the virtual articulation model can then be expressed as composite transforms of individual articulation transforms at various stages of interpolation.

The movement of the mandible from the closed pose to any of the other poses can be described, for each pose, as the combination of a rotation matrix (the composite of three rotations around the coordinate axes x, y, z) and a translation vector of the origin of coordinates. This combination (rotation plus translation vector) is usually called a "3D transformation matrix" or more narrowly a "rigid body transform."

In the particular case of human mandible movement, the possible movements are mechanically conditioned to the condyle and fossa, acting as a "ball joint." This particular condition of "ball joint" movements permits describing any of those mandible movements (coming from the different poses) as a unique pure rotation (without translation) instead of the combination of a rotation plus a translation (as any generic movement requires).

By moving the modified mandibular arch through various poses relative to the modified maxillary arch, virtual articulator 30 may determine contact points of the teeth at various states of treatment plans 26 (e.g., final target states and/or one or more intermediate states).

In one example, when determining contact points, virtual articulator 30 may additionally be further configured to predict wear facets at these contact points over time resulting from various mandibular motions, such as protrusive/retrusive and left/right lateral excursions.

In other examples, virtual articulator 30 may be configured to determine whether proper canine guidance is achieved. Virtual articulator 30 may be configured to make such a determination as a result of first contact occurring between upper and lower canines as the mandible is shifted laterally (i.e., lateral excursion), thus discluding the posterior teeth (i.e., opening the gape and removing contact between opposing teeth).

In other examples, virtual articulator 30 may be configured to determine whether proper anterior guidance is achieved. Virtual articulator 30 may be configured to make such a determination as a result of first contact occurring between upper and lower incisors as the mandible is protruded, thus discluding the posterior teeth (i.e., opening the gape and removing contact between opposing teeth).

Virtual articulation adds sophistication to the treatment planning frameworks. Some example dental treatment planning systems use static collision measurements given a current mandible and maxilla state of teeth to make determinations of the efficacy of the dental plan. In accordance with the techniques of this disclosure, dynamic collision module 32 may receive the contact points and other measurements produced by virtual articulator 30 and compute a dynamic collision metric. In general, dynamic collision module 32 may map the contact points from virtual articulator 32 and then additionally determine if the contact points are acceptable per clinical requirements. Dynamic collision module 32 may compute a dynamic collision metric based on what level of dynamic occlusion is acceptable.

In some examples, dynamic collision module 32 may compute a dynamic collision metric based on one or more of the following inputs received from virtual articulator 30. In general, dynamic collision module 32 may compute a dynamic collision metric based on contact measures from the various mandibular poses, including maximum intercuspation, lateral left excursion, lateral right excursion, protrusive, and retrusive.

In some examples, dynamic collision module 32 may determine a dynamic collision score based on the following factors. In maximum intercuspation (fully closed bite), a best score may be derived from a maximal number of contacts between opposing teeth, preferably all teeth and preferably a plurality of contacts per tooth. In lateral left excursion, ideally there is only one point of contact between upper and lower left canines; and one contact between upper and lower right canines for lateral right excursion. This single point of contact should exist through nearly the full range of motion from a displacement of slightly greater than the rest position at maximum intercuspation. For a protrusive excursion, there can be more than one point of contact between upper and lower incisors, preferably equal in number between left and right quadrants, and these contacts should be isolated to only the incisors through nearly the full range of motion from a displacement slightly greater than the rest position at maximum intercuspation. Additional contacts will reduce the quality of the dynamic occlusion and thus adversely affect the score.

In another example, dynamic collision module 32 may compute a dynamic collision metric based on a count of the number of collisions. A count of the number of collisions may include a count of collisions (unique colliding pairs of teeth) in a particular state (e.g., target final state and/or one or more intermediate states.

In another example, dynamic collision module 32 may compute a dynamic collision metric based on penetration depth. In one example, penetration depth may be the sum of penetration depths of all collisions in a particular state.

In another example, dynamic collision module 32 may compute a dynamic collision metric based on a collision contact points count. In one example, the collision contact points count is the total count of contact points in all collisions in a particular state. Collision contact points count may serve as an estimate of penetration since it will tend to increase as collisions get deeper.

In another example, dynamic collision module 32 may compute a dynamic collision metric based on a weighted sum of several of the metrics described above, e.g., α x Collision count + β x Penetration depth, where α and β are configurable weight values.

The geometric information discussed above can be used to provide physical information to inform treatment planning and facilitate effective communication with clinicians and patients. Scores may also be combined with other information, including landmarks, tooth movement between states, and tooth position to provide holistic oral health and comfort information. Such a system would go beyond being an orthodontic tool and rather serve as a unified treatment platform for dentists, orthodontists, and others.

In one example, a user may use the dynamic collision metric to determine a particular one of treatment plans 26 to use. In another example, a user may manually modify one or more of the intermediate and/or final states of treatment plans 26 based on the dynamic collision metric. In another example, virtual articulation system 24 may automatically determine a particular one of treatment plans 26 to use based on the dynamic collision metric. In another example, virtual articulation system 24 may automatically modify one or more of the intermediate and/or final states of treatment plans 26 based on the dynamic collision metric. For example, virtual articulation system 24 may choose the treatment plan that minimizes the dynamic collision metric as the final setup. In other examples, virtual articulation system 24 may be configured to output the selected treatment plan as a recommended/suggested treatment plan that the user can review and accept.

In addition to calculating a dynamic collision metric, virtual articulation system 24 may further determine a discomfort score based on the dynamic collision metric. Discomfort score module 34 may receive the dynamic collision metric from dynamic collision metric 32 and determine a discomfort score. The discomfort score may indicate the general level of discomfort a patient may experience given the quality and type of dynamic occlusion determined by the virtual articulation.

In one example, a user may use the discomfort score to determine a particular one of treatments plans 26 to use. In another example, a user may manually modify one or more of the intermediate and/or final states of treatment plans 26 based on the discomfort score. In another example, virtual articulation system 24 may automatically determine a particular one of treatments plans 26 to use based on the discomfort score. In another example, virtual articulation system 24 may automatically modify one or more of the intermediate and/or final states of treatment plans 26 based on the discomfort score. For example, virtual articulation system 24 may choose the treatment plan that minimizes the discomfort score as the final setup. Using a discomfort score to make treatment planning decisions may improve patient compliance. In general, patients are less likely to comply with treatment plans that create more discomfort compared to plans that create less discomfort.

For intermediate states, as part of the intermediate staging algorithm which explores different intermediate stages to interpolate between initial (malocclusion) and final (setup) states, virtual articulation system 24 may be configured to evaluate each stage for dynamic occlusion using the virtual articulator and contact points can be mapped which in turn can be converted to a discomfort score for the patient. Since multiple treatment trajectories can be produced between malocclusion and setup, virtual articulation system 24 may determine the trajectory of least discomfort.

In other examples, a user or virtual articulation system 24 may be configured to make treatment planning decisions based on a combination of dynamic collision metrics and discomfort scores.

Virtual articulation system 24 may optionally include a bruxism score module 38. Bruxism score module 38 may receive the output of dynamic collision module 32 (e.g., the dynamic collision metrics) and determine the likelihood of bruxing at any of the stages of treatment plans 26. Bruxing is a condition where a patient may grind, clench, and/or gnash their teeth. The dynamic collision metrics may indicate premature contacts between posterior teeth, i.e., a lack of canine and/or anterior guidance. Such lack of canine and/or anterior guidance may lead to discomfort, but also may tend to lead to a bruxing habit that results in significant tooth wear and/or facet creation. Tooth wear and facet creation may lead to a host of other pathologies, such as dental caries, chips, cracks, gingival recession, infection, and tooth loss.

Bruxism module 38 may receive the dynamic collision metric described above, along with dynamic measures of relevant metrics (e.g., class relationship of canines, mesial step of molars) to determine. Bruxism module 38 may determine a value (e.g., score) that is computed from such dynamic measures, where the score indicates an increased risk factor for tooth grinding during intermediate stages and/or final stage of treatment plans 26. An orthodontist may use this bruxing score to select and/or modify treatment plans 26. Additionally, data capturing intermediate bite configurations and the appearance or resolution of bruxism could be used to identify non-optimal bite configurations for the patient in question. For example, if an aesthetically preferable configuration is correlated to bruxism, a modified treatment can be proposed by the orthodontist, or if an intermediate bite configuration is correlated to bruxism, an alternative treatment path can be selected.

Based on the dynamic collision metrics and/or discomfort scores, virtual articulation system 24 may also determine one or more digital setups 36 that specify the form of various treatment modalities (e.g., clear tray aligners). In one example, virtual articulation system 24 may be configured to determine an exterior form of an aligner based on at least one of dynamic collision metric or the discomfort score. Typically, the exterior form of the aligners is manufactured to match to the teeth below the form. Because teeth are moving during treatment, they may move through a state where the actual teeth would generate discomfort. However, the external part of the aligner does not have to follow the shape of the teeth. Virtual articulation system 24 may output digital setups 36 that include plans and/or instructions for modifying the external aligner, given the virtual articulation, such that the upper and lower bites of the external aligner match up even when the teeth within the aligner would not line up. This would allow virtual articulation system 24 to find the "fastest" path to the final setup even if some of the intermediate positions would generate some discomfort because of misalignment.

In addition to the above techniques, virtual articulation system 24 may also include one or more user interface features where various aspects of the virtual articulation, dynamic collision metrics, and/or discomfort scores are displayed to a user on display device 16. Virtual articulation system may be configured to output and display data indicative of the dynamic collision metric and/or discomfort score for each of the one or more treatment plans. This data could be visual in nature, such as color-coding of the contact points or areas to indicate severity of discomfort. For example, contacts that are closer to the hinge axis of the condyles (e.g., in the temporomandibular joint (TMJ), i.e., more distal or more posterior) are likely to result in greater discomfort than those that are further from the hinge axis, both for reasons of neurology and for basic reasons of increased mechanical leverage and thus force or pressure given the same input force from the masseter muscles. Virtual articulation system 24 may use different colors that indicate the severity of the collision and/or potential discomfort (e.g., red for high discomfort, yellow for medium discomfort, green for low discomfort).

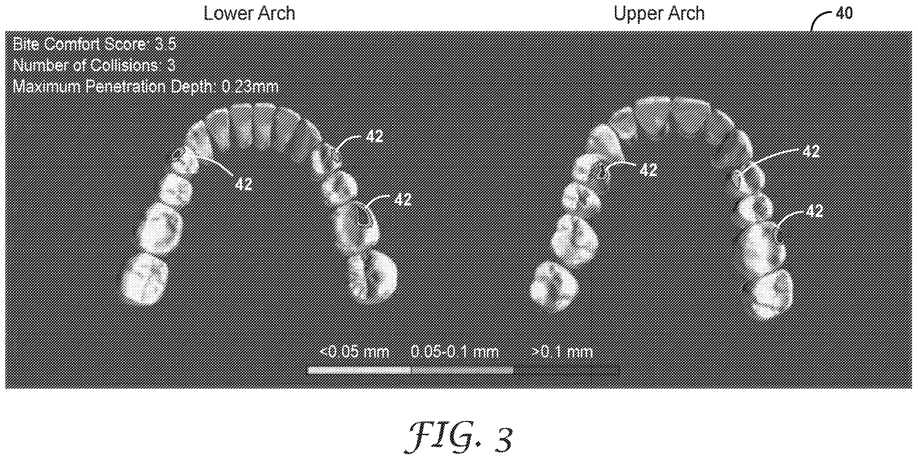

The user interface features described below enable user experience options for the orthodontist to visually evaluate a given state either by looking at the contact maps or looking at a simulation of the virtual articulation. This information can also be shared with a restorative or prosthodontic dentist for combined treatment plans. FIG. 3 illustrates an example user interface showing contact maps. As shown in FIG. 3, user interface 40 may include a visual display of contact points 42 on the lower arch (e.g., virtual mandibular arch) and the upper arch (e.g., virtual maxillary arch) of a patient. As shown in FIG. 3, contact points 42 are color coded in grayscale based on the amount of penetration of contact. The lightest gray color indicates less than 0.05 mm penetration. The medium gray color indicates penetration between 0.05 mm and 0.1 mm. The darkest gray color indicates penetration greater than 0.1 mm. Of course, rather than using gray-scale, the "heat map" of penetration depths may also be visually depicted with different colors. User interface 40 also displays a bite comfort score, the number of collisions, and the maximum penetration depth.

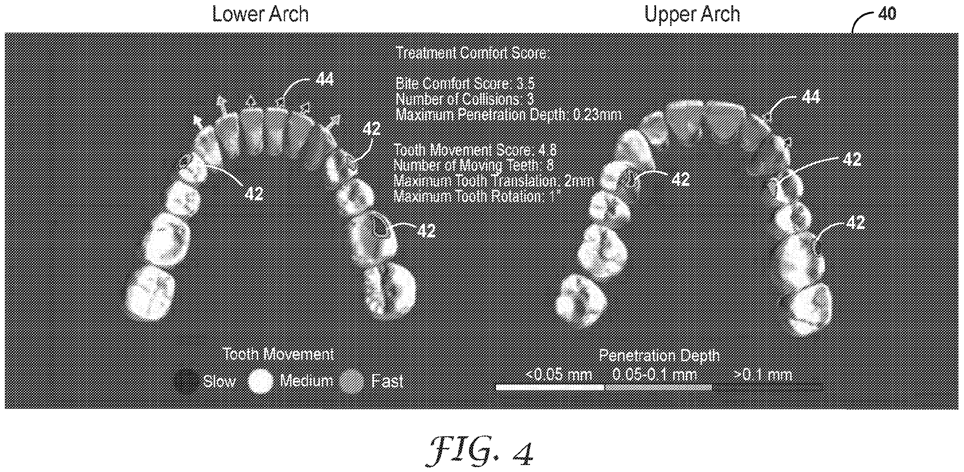

FIG. 4 illustrates another example user interface showing contact maps with teeth movement. User interface 40 in FIG. 4 further includes arrows 44 that indicate the speed of teeth movement at a particular state of a treatment plan. In the example of FIG. 4, slow teeth movement is indicated with a dark gray color, a medium tooth movement is shown with a medium gray color, and a fast tooth movement is shown with a light gray color. Again, rather than using gray-scale, the "heat map" of tooth movement may also be visually depicted with different colors. User interface 40 of FIG. 4 may further display a tooth movement score, a number of moving teeth, a maximum tooth translation, and a maximum tooth rotation.

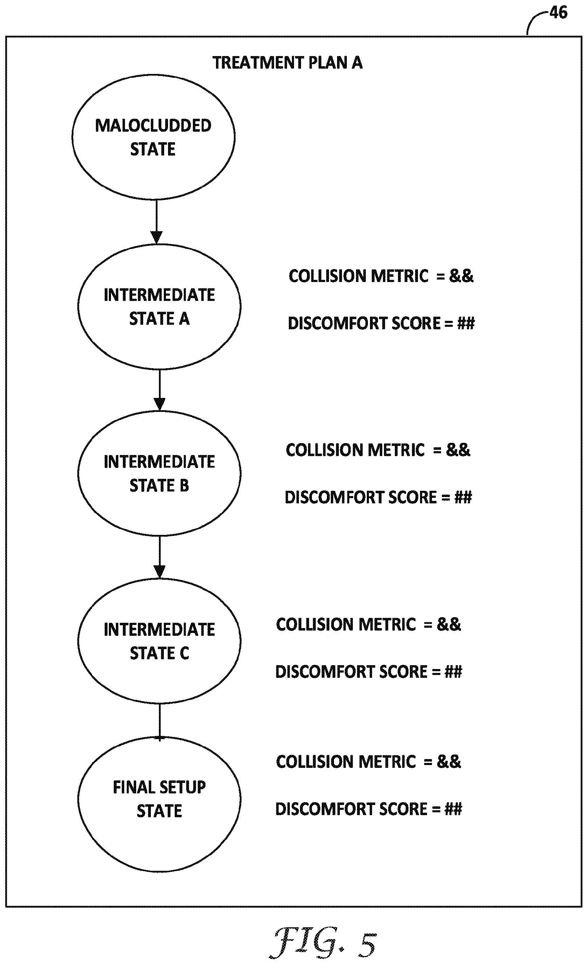

FIG. 5 illustrates another example user interface showing collision metrics and discomfort scores at various states. In particular, FIG. 5 illustrates an example user interface 46 that displays collision metrics and discomfort scores at each state of a treatment plan A. Rather than merely displaying text that indicates each treatment plan, in some examples, user interface 46 may display images depicting the modified arches at each of the states. In some examples, each of the states may be selectable by the user. A user may then make modification to one or more of the states. Based on the modification, virtual articulation system 24 may be configured to modify the virtual arches, re-run virtual articulation on the modified arches, perform metrics computation, and display any updated collision metrics and/or discomfort scores.

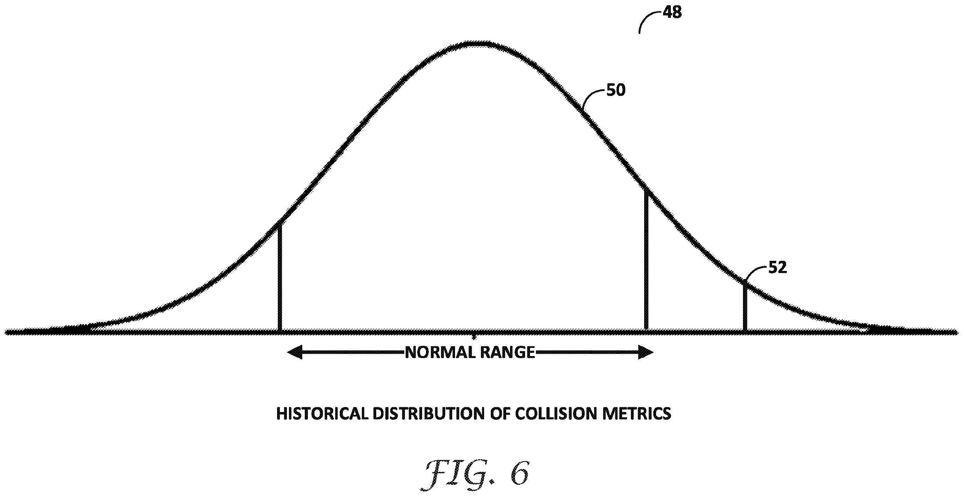

In other examples, virtual articulation system 24 may be further configured to display comparison of contact maps, virtual articulation, dynamic collision metrics, and/or comfort scores to: (1) past scans acquired from the same patient, and/or (2) average values generated from population data. For contact maps and virtual articulation, virtual articulation system 24 may be configured to display this comparison as a side-by-side view or an overlay. For comfort scores, virtual articulation system 24 may display a single patient's current and historic scores as part of a distribution of scores across population data. FIG. 6 illustrates another example user interface comparing a patient's metrics with population data metrics. In FIG. 6, virtual articulation system 24 may display a user interface 48 that displays a historical distribution of collision metrics 50 for a population of patient data. In addition, user interface 48 may display a current collision metric 52 for a current patient. In this way, a user may compare the collision metric of a patient to historical data. In the example of FIG. 6, the current collision metric 52 is outside of a normal range for that metric.

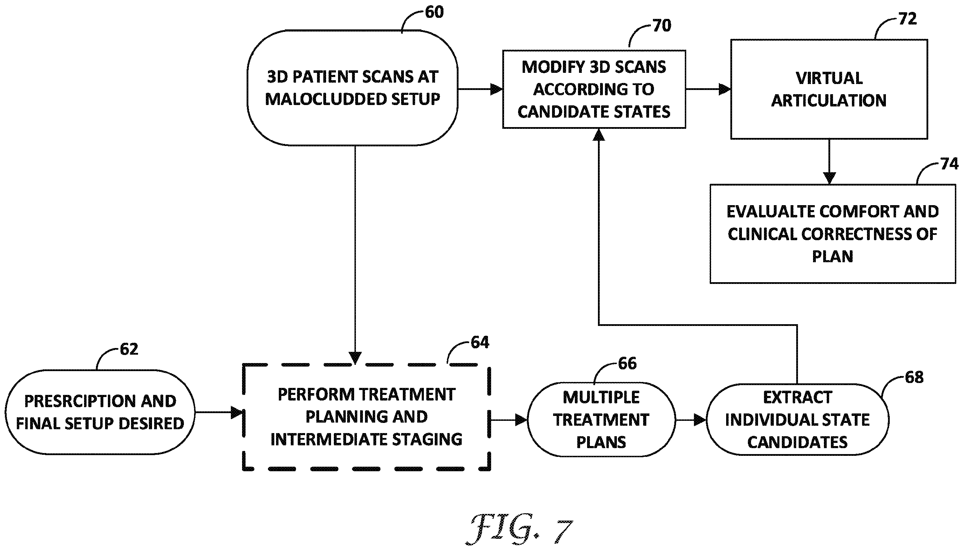

FIG. 7 is flow diagram showing one example process using the techniques of this disclosure. In particular, FIG. 7 shows a process from the viewpoint of a user (e.g., an orthodontist). First, the orthodontist may obtain 3D patient scans of the mandibular and maxillary arches of a patient, for example, at a maloccluded set (60). Based on the current state of the patient's teeth, the orthodontist may determine a prescription for treatment and a final setup state of the teeth that are desired (62). Optionally, the orthodontist may perform treatment planning and intermediate staging based on one or more desired final setup states (64). In some examples, virtual articulation system 24 may be configured to automatically generate one or more treatment plans with intermediate states based on desired final setup states.

Regardless of whether the treatment plans include only desired final setup or final setups along with suggested intermediate states, virtual articulation system 24 may be configured to analyze multiple treatment plans (66). The orthodontist may enter the multiple treatment plans 66 into virtual articulation system 24 (see FIG. 1). Virtual articulation system may be configured to extract individual state candidates (e.g., final setup states and/or intermediate states) from each of multiple treatment plans 66 (68). Virtual articulation system 24 may then modify the 3D scans according to the candidates states in the manner described above (70). Virtual articulation system 24 may then perform virtual articulation and computation of collision and discomfort scores, as described above (72). Virtual articulation system 24 may then display the results to the orthodontist in one or more of the manners described above. The orthodontist may then evaluate the comfort and/or clinical correction of the treatment plans (74).

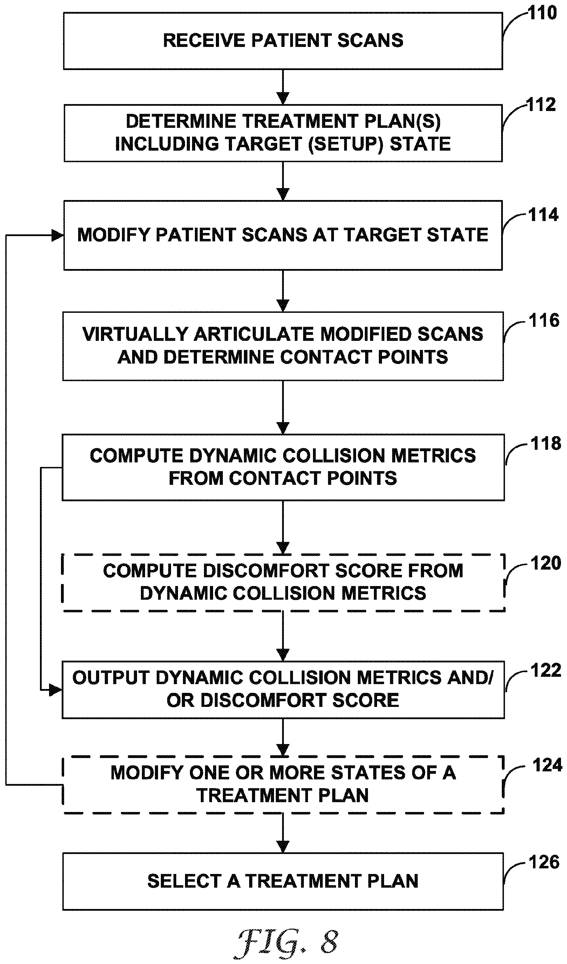

FIG. 8 is a flowchart illustrating techniques performed by virtual articulation system 24 according to the techniques of this disclosure. Virtual articulation system 24 may be configured to perform any combination of the techniques described above. Virtual articulation system 24 may be configured to receive patient scans (110). For example, virtual articulation system 24 may be configured to receive data indicative of a scan of a virtual maxillary arch representing a maxillary arch of a patient and a virtual mandibular arch representing a mandibular arch of the patient. In one example, the maxillary arch and the mandibular arch are in a maloccluded state.

Virtual articulation system 24 may be further configured to determine treatment plan(s), where the treatment plans include at least a target (e.g., setup) state (112). In some examples, the treatment plans do not yet have a target state. Instead, one or more target states may be tested using virtual articulation system 24. In some examples, virtual articulation system 24 may be configured to receive the treatment plans from a user (e.g., an orthodontist). In other examples, virtual articulation system 24 may be configured to determine one or more treatment plans for the patient based on an initial state of the virtual maxillary arch and the virtual mandibular arch and a target state for the virtual maxillary arch and the virtual mandibular arch.

Virtual articulation system 24 may be further configured to modify the patient scans at the target state (114). For example, virtual articulation system 24 may be configured to modify the virtual maxillary arch and the virtual mandibular arch at the target state for each of the one or more treatment plans. In some examples, virtual articulation system 24 may be configured to modify the virtual maxillary arch and the virtual mandibular arch to generate a target state for each of the one or more treatment plans.

Virtual articulation system 24 may be further configured to virtually articulate the modified scans and determine contact points of the patient's teeth (116). For example, virtual articulation system 24 may be configured to virtually articulate the modified virtual maxillary arch and the modified virtual mandibular arch to determine contact points at the target state for each of the one or more treatment plans. In one example, virtual articulation system 24 may virtually articulate the modified virtual maxillary arch and the modified virtual mandibular arch to determine contact points at one or more mandibular poses, wherein the one or more mandibular poses include a maximum intercuspation, a lateral left excursion, a lateral right excursion, a protrusive excursion, or a retrusive excursion.

In one example of the disclosure, through the virtual articulation, virtual articulation system 24 may be configured to predict wear facets at the contact points resulting from one or more mandibular motions. In another example, virtual articulation system 24 may be configured to determine whether proper canine guidance is achieved based on the virtual articulation. In another example, virtual articulation system 24 may be configured to determine whether proper anterior guidance is achieved based on the virtual articulation.

In another example, virtual articulation system 24 may be configured to virtually articulate the modified virtual maxillary arch and the modified virtual mandibular arch to determine penetration depth between contacting teeth. Virtual articulation system 24 may further predict an amount of facet wear based on the determined penetration depth.

Virtual articulation system 24 may be further configured to compute a dynamic collision metric based on the contact points at the target state for each of the one or more treatment plans (118). In one example, virtual articulation system 24 may compute the dynamic collision metric based on the contact points at one or more of the maximum intercuspation, the lateral left excursion, the lateral right excursion, the protrusive excursion, or the retrusive excursion.

Optionally, virtual articulation system 24 may further determine a discomfort score based on the dynamic collision metric at the target setup state for each of the one or more treatment plans (120). The discomfort score indicates a level of predicted discomfort in the patient.

Virtual articulation system 24 may further output data indicative of the discomfort score (if computed) for each of the one or more treatment plans, and output data indicative of the dynamic collision metric for each of the one or more treatment plans (122). In some examples, virtual articulation system 24 may display contact maps based on the contact points. In some examples, virtual articulation system 24 may display a simulation of the virtual articulation. In some examples, virtual articulation system 24 may display past scans of the patient alongside the virtual mandibular arch and the virtual maxillary arch. In some examples, virtual articulation system 24 may display at least one of the dynamic collision metric or the discomfort score alongside average values for a population of patients.

Optionally, through an automatic process or through user input, virtual articulation system 24 may modify one of the plurality of treatment plans based on at least one of the dynamic collision metric or the discomfort score (124). If so, virtual articulation system 24 may repeat process 114-122 based on the modified treatment plans.

Virtual articulation system 24 may further select a treatment plan, whether through user input or an automatic process (126). In one example, virtual articulation system 24 may determine a final setup state based on at least one of the dynamic collision metric or the discomfort score. In another example, virtual articulation system 24 may select one of the plurality of treatment plans based on at least one of the dynamic collision metric or the discomfort score.

In other examples, virtual articulation system 24 may determine a static collision metric of the virtual mandibular arch and the virtual maxillary arch and select one of the plurality of treatment plans based on the dynamic collision metric and the static collision metric.

The techniques of FIG. 8 were described with reference to virtually articulating and computing metrics on a final target state. However, the techniques of FIG. 8 may also be applied to one or more intermediate states of a treatment plan. For example, virtual articulation system 24 may be configured to modify the virtual maxillary arch and the virtual mandibular arch at each of the at least one intermediate state and the target state for each of the one or more treatment plans, virtually articulate the modified virtual maxillary arch and the modified virtual mandibular arch to determine contact points at each of the at least one intermediate state and the target state for each of the one or more treatment plans. Virtual articulation system 24 may further compute a dynamic collision metric based on the contact points at each of the at least one intermediate state and the target state for each of the one or more treatment plans and output data indicative of the dynamic collision metric for each of the at least one intermediate state and the target state for each of the one or more treatment plans. Likewise, virtual articulation system may modify one or more intermediate stages based on the dynamic collision metric and/or determine one or more intermediate states based on the dynamic collision metric.

## Claims

1. A method comprising:
Receiving (110), by a computing device (10), data (12) indicative of a scan of a virtual maxillary arch representing a maxillary arch of a patient and a virtual mandibular arch representing a mandibular arch of the patient;
Determining (112), by the computing device, one or more treatment plans (26) for the patient based on an initial state of the virtual maxillary arch and the virtual mandibular arch;
Modifying (114), by the computing device, the virtual maxillary arch and the virtual mandibular arch to generate a target state for each of the one or more treatment plans;
virtually articulating (116), by the computing device, the modified virtual maxillary arch and the modified virtual mandibular arch to determine contact points (42) at the target state for each of the one or more treatment plans;
computing (118), by the computing device, a dynamic collision metric based on the contact points at the target state for each of the one or more treatment plans;
determining (120), by the computing device, a discomfort score based on the dynamic collision metric at the target setup state for each of the one or more treatment plans, wherein the discomfort score indicates a level of predicted discomfort in the patient;
outputting (122), by the computing device, data indicative of the dynamic collision metric for each of the one or more treatment plans; and
outputting, by the computing device, data indicative of the discomfort score for each of the one or more treatment plans.

2. The method of claim 1, wherein determining (112) the one or more treatment plans (26) for the patient based on an initial state of the virtual maxillary arch and the virtual mandibular arch comprises determining that, in the initial state, the maxillary arch and the mandibular arch are in a maloccluded state.

3. The method of claim 1, further comprising:
predicting, by the computing device (10), wear facets at the contact points (42) resulting from one or more mandibular motions, wherein the one or more mandibular motions include one or more of a protrusive excursion, a retrusive excursion, a left lateral excursion, or a right lateral excursion.

4. The method of claim 1, further comprising:
determining, by the computing device (10), whether proper canine guidance is achieved based on the virtual articulation.

5. The method of claim 1, further comprising:
determining, by the computing device (10), whether proper anterior guidance is achieved based on the virtual articulation.

6. The method of claim 1, wherein virtually articulating (116), by the computing device (10), the modified virtual maxillary arch and the modified virtual mandibular arch comprises:
virtually articulating, by the computing device, the modified virtual maxillary arch and the modified virtual mandibular arch to determine contact points (42) at one or more mandibular poses, wherein the one or more mandibular poses include a maximum intercuspation, a lateral left excursion, a lateral right excursion, a protrusive excursion, or a retrusive excursion.

7. The method of claim 6, wherein computing, by the computing device (10), the dynamic collision metric based on the contact points (42) comprises:
Computing (118), by the computing device, the dynamic collision metric based on the contact points at one or more of the maximum intercuspation, the lateral left excursion, the lateral right excursion, the protrusive excursion, or the retrusive excursion.

8. The method of claim 1, wherein virtually articulating (116) further comprises:
virtually articulating, by the computing device (10), the modified virtual maxillary arch and the modified virtual mandibular arch to determine penetration depth between contacting teeth, the method further comprising:
predicting an amount of facet wear based on the determined penetration depth.

9. The method of claim 1, wherein the one or more treatment plans (26) comprise a plurality of treatment plans, the method further comprising:
selecting (126) one of the plurality of treatment plans based on at least one of the dynamic collision metric or the discomfort score.

10. The method of claim 9, the method further comprising:
determining a static collision metric of the virtual mandibular arch and the virtual maxillary arch; and
selecting (126) one of the plurality of treatment plans (26) based on the dynamic collision metric and the static collision metric.

11. The method of any combination of claims 1-10, wherein the one or more treatment plans (26) further include at least one intermediate state, the method further comprising:
modifying, by the computing device (10), the virtual maxillary arch and the virtual mandibular arch at each of the at least one intermediate state and the target state for each of the one or more treatment plans (26);
virtually articulating, by the computing device, the modified virtual maxillary arch and the modified virtual mandibular arch to determine contact points (42) at each of the at least one intermediate state and the target state for each of the one or more treatment plans;
computing, by the computing device, a dynamic collision metric based on the contact points at each of the at least one intermediate state and the target state for each of the one or more treatment plans;
outputting, by the computing device, data indicative of the dynamic collision metric for each of the at least one intermediate state and the target state for each of the one or more treatment plans.

12. An apparatus comprising:
a memory (22) configured to store data (12) indicative of a scan of a virtual maxillary arch representing a maxillary arch of a patient and a virtual mandibular arch representing a mandibular arch of the patient; and
a processor (20) in communication with the memory, the processor configured to:
determine (112) one or more treatment plans (26) for the patient based on an initial state of the virtual maxillary arch and the virtual mandibular arch;
modify (114) the virtual maxillary arch and the virtual mandibular arch to generate a target state for each of the one or more treatment plans;
virtually articulate (116) the modified virtual maxillary arch and the modified virtual mandibular arch to determine contact points (42) at the target state for each of the one or more treatment plans;
compute (118) a dynamic collision metric based on the contact points at the target state for each of the one or more treatment plans;
determine (120) a discomfort score based on the dynamic collision metric at the target setup state for each of the one or more treatment plans, wherein the discomfort score indicates a level of predicted discomfort in the patient;
output (122) data indicative of the dynamic collision metric for each of the one or more treatment plans; and
output data indicative of the discomfort score for each of the one or more treatment plans.

13. The apparatus of claim 12, wherein the processor (20) is further configured to:
predict wear facets at the contact points (42) resulting from one or more mandibular motions.

14. The apparatus of claim 12, wherein the processor (20) is further configured to:
determine whether proper canine guidance is achieved based on the virtual articulation.

15. The apparatus of claim 14, wherein to virtually articulate (116) the modified virtual maxillary arch and the modified virtual mandibular arch, the processor (20) is further configured to:
virtually articulate the modified virtual maxillary arch and the modified virtual mandibular arch to determine contact points (42) at one or more mandibular poses, wherein the one or more mandibular poses include a maximum intercuspation, a lateral left excursion, a lateral right excursion, a protrusive excursion, or a retrusive excursion.

## Patentansprüche

1. Ein Verfahren, aufweisend:
Empfangen (110), durch eine Rechenvorrichtung (10), von Daten (12), die einen Scan eines virtuellen Oberkieferbogens, der einen Oberkieferbogen eines Patienten darstellt, und eines virtuellen Unterkieferbogens, der einen Unterkieferbogen des Patienten darstellt, angeben;
Bestimmen (112), durch die Rechenvorrichtung, eines oder mehrerer Behandlungspläne (26) für den Patienten basierend auf einem Anfangszustand des virtuellen Oberkieferbogens und des virtuellen Unterkieferbogens;
Modifizieren (114), durch die Rechenvorrichtung, des virtuellen Oberkieferbogens und des virtuellen Unterkieferbogens, um einen Zielzustand für jeden des einen oder der mehreren Behandlungspläne zu erzeugen;
virtuelles Artikulieren (116), durch die Rechenvorrichtung, des modifizierten virtuellen Oberkieferbogens und des modifizierten virtuellen Unterkieferbogens, um Kontaktpunkte (42) in dem Zielzustand für jeden des einen oder der mehreren Behandlungspläne zu bestimmen;
Berechnen (118), durch die Rechenvorrichtung, einer dynamischen Kollisionsmetrik basierend auf den Kontaktpunkten in dem Zielzustand für jeden des einen oder der mehreren Behandlungspläne;
Bestimmen (120), durch die Rechenvorrichtung, einer Bewertung von Beschwerden basierend auf der dynamischen Kollisionsmetrik in dem Zielanordnungszustand für jeden des einen oder der mehreren Behandlungspläne, wobei die Bewertung von Beschwerden einen Grad an vorhergesagten Beschwerden bei dem Patienten angibt;
Ausgeben (122), durch die Rechenvorrichtung, von Daten, die die dynamische Kollisionsmetrik für jeden des einen oder der mehreren Behandlungspläne angeben; und
Ausgeben, durch die Rechenvorrichtung, von Daten, die die Bewertung von Beschwerden für jeden des einen oder der mehreren Behandlungspläne angeben.

2. Das Verfahren nach Anspruch 1, wobei das Bestimmen (112) des einen oder der mehreren Behandlungspläne (26) für den Patienten basierend auf einem Anfangszustand des virtuellen Oberkieferbogens und des virtuellen Unterkieferbogens ein Bestimmen, dass, in dem Anfangszustand, der Oberkieferbogen und der Unterkieferbogen in einem Malokklusionszustand sind, aufweist.

3. Das Verfahren nach Anspruch 1, ferner aufweisend:
Vorhersagen, durch die Rechenvorrichtung (10), von Abnutzungsfacetten an den Kontaktpunkten (42), die von einer oder mehreren Unterkieferbewegungen resultieren, wobei die eine oder die mehreren Unterkieferbewegungen eine oder mehrere von einer protrusiven Exkursion, einer retrusiven Exkursion, einer linken Lateralexkursion oder einer rechten Lateralexkursion einschließen.

4. Das Verfahren nach Anspruch 1, ferner aufweisend:
Bestimmen, durch die Rechenvorrichtung (10), ob eine geeignete Eckzahnführung basierend auf der virtuellen Artikulation erreicht wird.

5. Das Verfahren nach Anspruch 1, ferner aufweisend:
Bestimmen, durch die Rechenvorrichtung (10), ob eine geeignete Vorderzahnführung basierend auf der virtuellen Artikulation erreicht wird.

6. Das Verfahren nach Anspruch 1, wobei das virtuelle Artikulieren (116), durch die Rechenvorrichtung (10), des modifizierten virtuellen Oberkieferbogens und des modifizierten virtuellen Unterkieferbogens aufweist:
virtuelles Artikulieren, durch die Rechenvorrichtung, des modifizierten virtuellen Oberkieferbogens und des modifizierten virtuellen Unterkieferbogens, um Kontaktpunkte (42) an einer oder mehreren Unterkieferposen zu bestimmen, wobei die eine oder die mehreren Unterkieferposen eine maximale Schlussbissstellung, eine laterale Linksexkursion, eine laterale Rechtsexkursion, eine protrusive Exkursion oder eine retrusive Exkursion einschließen.

7. Das Verfahren nach Anspruch 6, wobei das Berechnen, durch die Rechenvorrichtung (10), der dynamischen Kollisionsmetrik basierend auf den Kontaktpunkten (42) aufweist:
Berechnen (118), durch die Rechenvorrichtung, der dynamischen Kollisionsmetrik basierend auf den Kontaktpunkten an einer oder mehreren von der maximalen Schlussbissstellung, der lateralen Linksexkursion, der lateralen Rechtsexkursion, der protrusiven Exkursion oder der retrusiven Exkursion.

8. Das Verfahren nach Anspruch 1, wobei das virtuelle Artikulieren (116) ferner aufweist:
virtuelles Artikulieren, durch die Rechenvorrichtung (10), des modifizierten virtuellen Oberkieferbogens und des modifizierten virtuellen Unterkieferbogens, um eine Eindringtiefe zwischen den Kontaktzähnen zu bestimmen, das Verfahren ferner aufweisend:
Vorhersagen einer Menge an Facettenabnutzung basierend auf der bestimmten Eindringtiefe.

9. Das Verfahren nach Anspruch 1, wobei der eine oder die mehreren Behandlungspläne (26) eine Mehrzahl von Behandlungsplänen aufweisen, das Verfahren ferner aufweisend:
Auswählen (126) eines der Mehrzahl von Behandlungsplänen basierend auf mindestens einer von der dynamischen Kollisionsmetrik oder der Bewertung von Beschwerden.

10. Das Verfahren nach Anspruch 9, das Verfahren ferner aufweisend:
Bestimmen einer statischen Kollisionsmetrik des virtuellen Unterkieferbogens und des virtuellen Oberkieferbogens; und
Auswählen (126) eines der Mehrzahl von Behandlungsplänen (26) basierend auf der dynamischen Kollisionsmetrik und der statischen Kollisionsmetrik.

11. Das Verfahren nach einer Kombination von Ansprüchen 1 bis 10, wobei der eine oder die mehreren Behandlungspläne (26) ferner mindestens einen Zwischenzustand einschließen, das Verfahren ferner aufweisend:
Modifizieren, durch die Rechenvorrichtung (10), des virtuellen Oberkieferbogens und des virtuellen Unterkieferbogens an jedem des mindestens einen Zwischenzustands und des Zielzustands für jeden des einen oder der mehreren Behandlungspläne (26);
virtuelles Artikulieren, durch die Rechenvorrichtung, des modifizierten virtuellen Oberkieferbogens und des modifizierten virtuellen Unterkieferbogens, um Kontaktpunkte (42) in jedem des mindestens einen Zwischenzustands und des Zielzustands für jeden des einen oder der mehreren Behandlungspläne zu bestimmen;
Berechnen, durch die Rechenvorrichtung, einer dynamischen Kollisionsmetrik basierend auf den Kontaktpunkten in jedem des mindestens einen Zwischenzustands und des Zielzustands für jeden des einen oder der mehreren Behandlungspläne;
Ausgeben, durch die Rechenvorrichtung, von Daten, die die dynamische Kollisionsmetrik für jeden des mindestens einen Zwischenzustands und des Zielzustands für jeden des einen oder der mehreren Behandlungspläne angeben.

12. Eine Einrichtung, aufweisend:
einen Speicher (22), der konfiguriert ist, um Daten (12) abzulegen, die einen Scan eines virtuellen Oberkieferbogens, der einen Oberkieferbogen eines Patienten darstellt, und eines virtuellen Unterkieferbogens, der einen Unterkieferbogen des Patienten darstellt, angeben; und
einen Prozessor (20) in Kommunikation mit dem Speicher, wobei der Prozessor konfiguriert ist zum:
Bestimmen (112) eines oder mehrerer Behandlungspläne (26) für den Patienten basierend auf einem Anfangszustand des virtuellen Oberkieferbogens und des virtuellen Unterkieferbogens;
Modifizieren (114) des virtuellen Oberkieferbogens und des virtuellen Unterkieferbogens, um einen Zielzustand für jeden des einen oder der mehreren Behandlungspläne zu erzeugen;
virtuelles Artikulieren (116) des modifizierten virtuellen Oberkieferbogens und des modifizierten virtuellen Unterkieferbogens, um Kontaktpunkte (42) in dem Zielzustand für jeden des einen oder der mehreren Behandlungspläne zu bestimmen;
Berechnen (118) einer dynamischen Kollisionsmetrik basierend auf den Kontaktpunkten in dem Zielzustand für jeden des einen oder der mehreren Behandlungspläne;
Bestimmen (120) einer Bewertung von Beschwerden basierend auf der dynamischen Kollisionsmetrik in dem Zielanordnungszustand für jeden des einen oder der mehreren Behandlungspläne, wobei die Bewertung von Beschwerden einen Grad an vorhergesagten Beschwerden bei dem Patienten angibt;
Ausgeben (122) von Daten, die die dynamische Kollisionsmetrik für jeden des einen oder der mehreren Behandlungspläne angeben; und
Ausgeben von Daten, die die Bewertung von Beschwerden für jeden des einen oder der mehreren Behandlungspläne angeben.

13. Die Einrichtung nach Anspruch 12, wobei der Prozessor (20) ferner konfiguriert ist zum:
Vorhersagen von Abnutzungsfacetten an den Kontaktpunkten (42), die von einer oder mehreren Unterkieferbewegungen resultieren.

14. Die Einrichtung nach Anspruch 12, wobei der Prozessor (20) ferner konfiguriert ist zum:
Bestimmen, ob eine geeignete Eckzahnführung basierend auf der virtuellen Artikulation erreicht wird.

15. Die Einrichtung nach Anspruch 14, wobei zum virtuellen Artikulieren (116) des modifizierten virtuellen Oberkieferbogens und des modifizierten virtuellen Unterkieferbogens, der Prozessor (20) ferner konfiguriert ist zum:
virtuellen Artikulieren des modifizierten virtuellen Oberkieferbogens und des modifizierten virtuellen Unterkieferbogens, um Kontaktpunkte (42) an einer oder mehreren Unterkieferposen zu bestimmen, wobei die eine oder die mehreren Unterkieferposen eine maximale Schlussbissstellung, eine laterale Linksexkursion, eine laterale Rechtsexkursion, eine protrusive Exkursion oder eine retrusive Exkursion einschließen.

## Revendications

1. Procédé comprenant :
la réception (110), par un dispositif de calcul (10), de données (12) indicatives d'un balayage d'une arcade maxillaire virtuelle représentant une arcade maxillaire d'un patient et d'une arcade mandibulaire virtuelle représentant une arcade mandibulaire du patient ;
la détermination (112), par le dispositif de calcul, d'un ou plusieurs plans de traitement (26) pour le patient en fonction d'un état initial de l'arcade maxillaire virtuelle et de l'arcade mandibulaire virtuelle ;
la modification (114), par le dispositif de calcul, de l'arcade maxillaire virtuelle et de l'arcade mandibulaire virtuelle pour générer un état cible pour chacun des un ou plusieurs plans de traitement ;
l'articulation virtuelle (116), par le dispositif de calcul, de l'arcade maxillaire virtuelle modifiée et de l'arcade mandibulaire virtuelle modifiée pour déterminer des points de contact (42) à l'état cible pour chacun des un ou plusieurs plans de traitement ;
le calcul (118), par le dispositif de calcul, d'un paramètre de collision dynamique en fonction des points de contact à l'état cible pour chacun des un ou plusieurs plans de traitement ;
la détermination (120), par le dispositif de calcul, d'un score d'inconfort en fonction du paramètre de collision dynamique à l'état réglé cible pour chacun des un ou plusieurs plans de traitement, dans lequel le score d'inconfort indique un niveau d'inconfort prédit chez le patient ;
la sortie (122), par le dispositif de calcul, de données indicatives du paramètre de collision dynamique pour chacun des un ou plusieurs plans de traitement ; et
la sortie, par le dispositif de calcul, de données indicatives du score d'inconfort pour chacun des un ou plusieurs plans de traitement.

2. Procédé selon la revendication 1, dans lequel la détermination (112) de l'un ou plusieurs plans de traitement (26) pour le patient en fonction d'un état initial de l'arcade maxillaire virtuelle et de l'arcade mandibulaire virtuelle comprend la détermination que, dans l'état initial, l'arcade maxillaire et l'arcade mandibulaire sont dans un état de malocclusion.

3. Procédé selon la revendication 1, comprenant en outre :
la prédiction, par le dispositif de calcul (10), de facettes d'usure au niveau des points de contact (42) résultant d'un ou plusieurs mouvements mandibulaires, dans lequel l'un ou plusieurs mouvements mandibulaires comportent une ou plusieurs d'une excursion en propulsion, d'une excursion en rétropulsion, d'une excursion latérale gauche, ou d'une excursion latérale droite.

4. Procédé selon la revendication 1, comprenant en outre :
la détermination, par le dispositif de calcul (10), du fait de savoir si un guidage de canine correct est réalisé en fonction de l'articulation virtuelle.

5. Procédé selon la revendication 1, comprenant en outre :
la détermination, par le dispositif de calcul (10), du fait de savoir si un guidage antérieur correct est réalisé en fonction de l'articulation virtuelle.

6. Procédé selon la revendication 1, dans lequel l'articulation virtuelle (116), par le dispositif de calcul (10), de l'arcade maxillaire virtuelle modifiée et de l'arcade mandibulaire virtuelle modifiée comprend :
l'articulation virtuelle, par le dispositif de calcul, de l'arcade maxillaire virtuelle modifiée et de l'arcade mandibulaire virtuelle modifiée pour déterminer des points de contact (42) au niveau d'une ou plusieurs poses mandibulaires, dans lequel l'une ou plusieurs poses mandibulaires comportent une intercuspidation maximale, une excursion latérale gauche, une excursion latérale droite, une excursion en propulsion, ou une excursion en rétropulsion.

7. Procédé selon la revendication 6, dans lequel le calcul, par le dispositif de calcul (10), du paramètre de collision dynamique en fonction des points de contact (42) comprend :
le calcul (118), par le dispositif de calcul, du paramètre de collision dynamique en fonction des points de contact au niveau d'une ou plusieurs de l'intercuspidation maximale, de l'excursion latérale gauche, de l'excursion latérale droite, de l'excursion en propulsion, ou de l'excursion en rétropulsion.

8. Procédé selon la revendication 1, dans lequel l'articulation virtuelle (116) comprend en outre :
l'articulation virtuelle, par le dispositif de calcul (10), de l'arcade maxillaire virtuelle modifiée et de l'arcade mandibulaire virtuelle modifiée pour déterminer la profondeur de pénétration entre des dents en contact, le procédé comprenant en outre :
la prédiction d'une quantité d'usure de facette en fonction de la profondeur de pénétration déterminée.

9. Procédé selon la revendication 1, dans lequel l'un ou plusieurs plans de traitement (26) comprennent une pluralité de plans de traitement, le procédé comprenant en outre :
la sélection (126) de l'un de la pluralité de plans de traitement en fonction d'au moins l'un du paramètre de collision dynamique ou du score d'inconfort.

10. Procédé selon la revendication 9, le procédé comprenant en outre :
la détermination d'un paramètre de collision statique de l'arcade mandibulaire virtuelle et de l'arcade maxillaire virtuelle ; et
la sélection (126) de l'un de la pluralité de plans de traitement (26) en fonction du paramètre de collision dynamique et du paramètre de collision statique.

11. Procédé selon l'une quelconque combinaison des revendications 1 à 10, dans lequel l'un ou plusieurs plans de traitement (26) comportent en outre au moins un état intermédiaire, le procédé comprenant en outre :
la modification, par le dispositif de calcul (10), de l'arcade maxillaire virtuelle et de l'arcade mandibulaire virtuelle à chacun de l'au moins un état intermédiaire et de l'état cible pour chacun des un ou plusieurs plans de traitement (26) ;
l'articulation virtuelle, par le dispositif de calcul, de l'arcade maxillaire virtuelle modifiée et de l'arcade mandibulaire virtuelle modifiée pour déterminer des points de contact (42) à chacun de l'au moins un état intermédiaire et de l'état cible pour chacun des un ou plusieurs plans de traitement ;
le calcul, par le dispositif de calcul, d'un paramètre de collision dynamique en fonction des points de contact à chacun de l'au moins un état intermédiaire et de l'état cible pour chacun des un ou plusieurs plans de traitement ;
la sortie, par le dispositif de calcul, de données indicatives du paramètre de collision dynamique pour chacun de l'au moins un état intermédiaire et de l'état cible pour chacun des un ou plusieurs plans de traitement.

12. Appareil comprenant :
une mémoire (22) configurée pour stocker des données (12) indicatives d'un balayage d'une arcade maxillaire virtuelle représentant une arcade maxillaire d'un patient et d'une arcade mandibulaire virtuelle représentant une arcade mandibulaire du patient ; et
un processeur (20) en communication avec la mémoire, le processeur étant configuré pour :
déterminer (112) un ou plusieurs plans de traitement (26) pour le patient en fonction d'un état initial de l'arcade maxillaire virtuelle et de l'arcade mandibulaire virtuelle ;
modifier (114) l'arcade maxillaire virtuelle et l'arcade mandibulaire virtuelle pour générer un état cible pour chacun des un ou plusieurs plans de traitement ;
articuler virtuellement (116) l'arcade maxillaire virtuelle modifiée et l'arcade mandibulaire virtuelle modifiée pour déterminer des points de contact (42) à l'état cible pour chacun des un ou plusieurs plans de traitement ;
calculer (118) un paramètre de collision dynamique en fonction des points de contact à l'état cible pour chacun des un ou plusieurs plans de traitement ;
déterminer (120) un score d'inconfort en fonction du paramètre de collision dynamique à l'état réglé cible pour chacun des un ou plusieurs plans de traitement, dans lequel le score d'inconfort indique un niveau d'inconfort prédit chez le patient ;
sortir (122) des données indicatives du paramètre de collision dynamique pour chacun des un ou plusieurs plans de traitement ; et
sortir des données indicatives du score d'inconfort pour chacun des un ou plusieurs plans de traitement.

13. Appareil selon la revendication 12, dans lequel le processeur (20) est en outre configuré pour :
prédire des facettes d'usure au niveau des points de contact (42) résultant d'un ou plusieurs mouvements mandibulaires.

14. Appareil selon la revendication 12, dans lequel le processeur (20) est en outre configuré pour :
déterminer si un guidage de canine correct est réalisé en fonction de l'articulation virtuelle.

15. Appareil selon la revendication 14, dans lequel pour articuler virtuellement (116) l'arcade maxillaire virtuelle modifiée et l'arcade mandibulaire virtuelle modifiée, le processeur (20) est en outre configuré pour :
articuler virtuellement l'arcade maxillaire virtuelle modifiée et l'arcade mandibulaire virtuelle modifiée pour déterminer des points de contact (42) au niveau d'une ou plusieurs poses mandibulaires, dans lequel les une ou plusieurs poses mandibulaires comportent une intercuspidation maximale, une excursion latérale gauche, une excursion latérale droite, une excursion en propulsion, ou une excursion en rétropulsion.
